# FASCICULE DE BREVET EUROPEEN

(11) **EP 0 684 804 B1**
(45) Date de publication et mention de la délivrance du brevet: **17.05.2000**
(21) Numéro de dépôt: 93913162.9
(22) Date de dépôt: 18.06.1993
(51) Int. Cl.: A61F 2/38

(54) **PROTHESE TOTALE DU GENOU**
GANZKNIEPROTHESE
TOTAL KNEE PROSTHESIS

(30) Priorité: 19.06.1992 FR 9207843
(43) Date de publication de la demande: 06.12.1995
(73) Titulaire: Spike Trading Lda, Funchal, Madeira (PT)
(72) Inventeur: Buscayret, Christian, 34000 Montpellier (FR)
(74) Mandataire: Maccalli, Marco
(86) Numéro de dépôt international: FR9300608
(87) Numéro de publication internationale: WO9400081

(56) Documents cités:
- EP-A- 0 119 394
- EP-A- 0 410 237
- DE-A- 3 119 841
- FR-A- 2 601 873
- FR-A- 2 628 316
- FR-A- 2 635 679
- GB-A- 1 328 497
- US-A- 4 822 362

## Description

La présente invention concerne une endoprothèse totale d'articulation du genou.

Il existe de nombreuses prothèses de genou, autorisant la flexion/extension autour d'un axe horizontal et le pivotement interne et externe du tibia autour d'un axe vertical, décrites par exemple par les brevets FR N°2 287 895, N°2 330 375, N°2 612 767, N° 2 405 064, N° 2 601 873, N° 2 167 381, CH N° 568 066 ou US N°4 865 606.

Le document EP-A-0 410 237 décrit une prothèse totale du genou selon les caractéristiques du préambule de la revendication 1, comprenant un implant tibial et un implant fémoral, l'implant tibial puvant pivoter par rapport à l'implant fémoral selon, d'une part, un axe sensiblement horizontal, afin de permettre le mouvement de flexion/extension de la jambe et, d'autre part, un axe vertical qui, permettant un pivotement limité du tibia selon son axe, est constitué par un pion monté avec possibilité de coulissement axial dans l'alésage qui lui sert de palier de rotation.
L'axe vertical selon lequel l'impiant tibial pivote est, d'une part, déporté vers l'avant par rapport à l'axe horizontal de flexion/extension de la jambe et, d'autre part, décalé vers l'intérieur par rapport à l'axe médian longitudinal de l'impiant tibial. La prothèse selon EP-A-0410237 comprend une pièce central d'assemblage des éléments tibial et fèmoral. La pièce réalise des moyens de guidage de l'élément tibial par rapport à l'élément fémoral selon deux axes non concourants.
Il apparaït, en pratique, que les prothèses de genou existantes ne respectent pas au mieux les fonctions cinématiques, dynamiques et tribologiques de articulation naturelle.
En outre, la stabilité de l'articulation prothétique au cours de son mouvement n'est pas parfaite.
Enfin, l'appui exercé par le poids du coprs est réparti essentiellement sur les surfaces condyliennes et glénoïdes, ce qui conduit à leur usure relativement rapide.

La présente invention vise à remédier à ces inconvénients.

En particulier, ces inconvénients sont résolus par une prothèse selon la revendication 1.

De toute façon, l'invention sera bien comprise à l'aide de la description qui suit, en référence au dessin schématique annexé représentant, à titre d'exemples non limitatifs, plusieurs formes de réalisation de la prothèse de genou qu'elle concerne.
La figure 1 en est une vue an coupe longitudinale antéro-postérieure éclatée, selon une première forme de réalisation ;
la figure 2 en est une vue similaire, montée ;
la figure 3 en est une vue, montée, en coupe longitudinale selon un plan de coupe perpendiculaire au plan de coupe selon les figures 1 et 2 ; et
la figure 4 est une vue en coupe longitudinale d'une variante de réalisation de l'implant tibial.

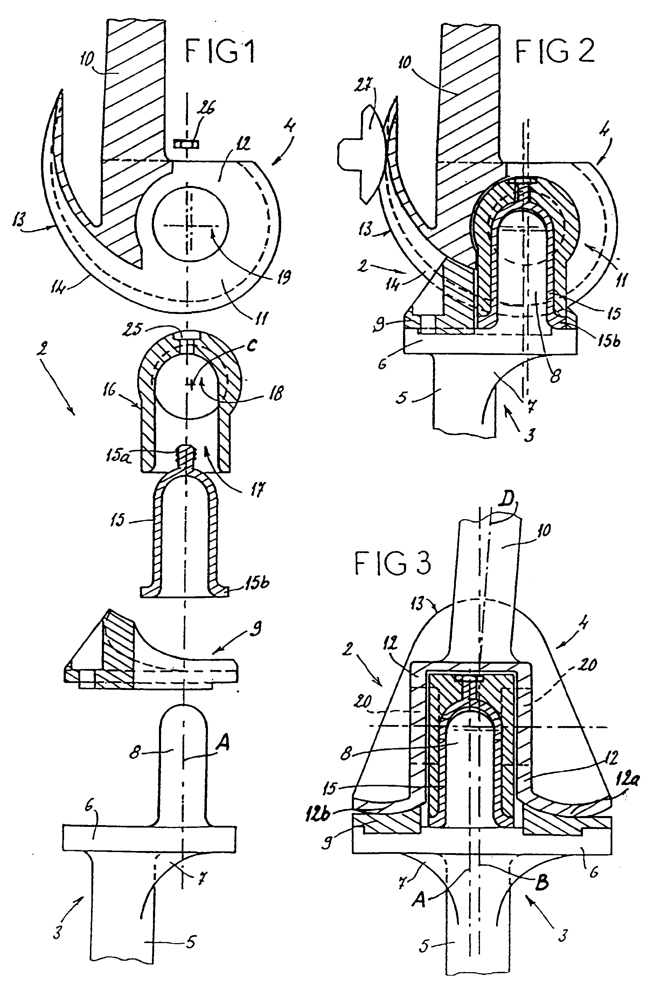

Les figures 1 à 3 représentent sous différents angles une première prothèse d'articulation de genou 2, comprenant un implant tibial 3 et un implant fémoral 4, dans laquelle l'implant tibial 3 peut pivoter par rapport à l'implant fémoral 4 selon, d'une part, un axe sensiblement horizontal, afin de permettre le mouvement de flexion/extension de la jambe et, d'autre part, un axe vertical permettant un pivotement limité du tibia selon son axe.

L'implant tibial 3 comprend une tige 5 pour son ancrage dans le canal médullaire du tibia, un plateau horizontal 6, dont la liaison avec la tige 5 est renforcée par deux nervures obliques 7 tournées vers l'arrière, un pion 8 d'axe vertical A, qui est déporté vers l'intérieur de la prothèse 2 par rapport à l'axe médian longitudinal B de l'implant tibial 3, et un insert 9 en matériau favorisant le glissement, tel qu'en polyéthylène à haute densité, qui est monté sur le plateau 6 par un assemblage à queue d'aronde. Le système à queue d'aronde peut être remplacé par un simple assemblage par emboîtement et vis.

La face supérieure des parties latérales de l'insert 9 délimite les surfaces glénoïdes de l'articulation, tandis que sa partie centrale, bombée, est destinée à coulisser dans la gorge trochléenne de l'implant fémoral 4.

L'implant fémoral comprend une tige 10 pour son ancrage dans le canal médullaire du fémur, une cavité centrale 11 en secteur de cercle, délimitée par deux parois latérales 12 et dont l'ouverture est prolongée par deux parois bombées 12a, 12b formant les surfaces condyliennes et un bouclier trochléen 13, dans lequel est aménagée la gorge trochléenne 14.

La prothèse 2 comprend également un fourreau 15 en matériau favorisant le glissement, destiné à être engagé sur le pion 8, et une pièce centrale d'assemblage 16, qui est percée d'une cavité borgne longitudinale 17, permettant son engagement sur le fourreau 15, avec mise en contact du fourreau contre la paroi de la cavité. La pièce d'assemblage 16 comprend deux alésages horizontaux coaxiaux 18 ménagés dans ses faces latérales. L'axe de la cavité 17, confondu avec l'axe du doigt 8, est déporté vers l'avant par rapport à l'axe C des alésages 18.

Le fourreau 15 comprend un téton axial fileté 15a destiné à être engagé au travers d'un trou lamé 25 aménagé dans la pièce 16 et à recevoir un écrou 26, pour son assemblage à la pièce 16.

Comme le montre la figure 2, les alésages 18 sont destinés à venir en coïncidence avec deux alésages 19 percés dans les parois latérales 12 et à recevoir deux tourillons coaxiaux 20 constituant l'axe de pivotement horizontal de l'implant tibial 3. Les tourillons 20 peuvent être assemblés à l'implant fémoral 4 par vissage ou emmanchement.

Selon une autre caractéristique de l'invention, l'axe D de la tige 10 de l'implant fémoral est incliné de haut en bas et de l'extérieur vers l'intérieur, en formant un angle de l'ordre de 4° avec l'axe longitudinal du corps de l'implant fémoral. En outre, la surface condylienne extérieure 12a est plus proéminente que la surface condylienne intérieure 12b, c'est-à-dire que celle 12a dépasse plus vers le bas que celle 12b.

Le pion 8 est libre en coulissement par rapport au fourreau 15 et à la pièce centrale d'assemblage 16, ce qui autorise un certain jeu axial de l'implant fémoral 4 par rapport à l'implant tibial 3, rapprochant le mouvement que permet la prothèse 2 de celui de l'articulation naturelle.

En outre, l'extrémité libre du pion 8 ne vient pas en appui contre le fond du fourreau 15 lorsque les surfaces condyliennes et glénoïdes sont en contact, mais le fourreau 15 comprend une collerette 15b du côté opposé à l'extrémité libre du pion 8, sur laquelle vient s'appuyer la pièce centrale d'assemblage 16 lorsque s'exerce l'appui en compression de l'implant fémoral 4 sur l'implant tibial 3. Ainsi, cet appui est réparti non seulement sur les surfaces condyliennes et glénoïdes mais également sur le fourreau 15, la pièce 16 et les tourillons 20. L'usure de ces surfaces est, par conséquent, réduite.

La prothèse 2 peut comprendre, en outre, un implant rotulien 27 rapporté sur la rotule à la place de la partie réséquée de celle-ci et formant un patin glissant le long du bouclier trochléen 13.

Les nervures 7 sont destinées à supporter directement les charges mécaniques de compression fémoro-tibiale appliquées au plateau tibial 6. Cette disposition permet de réduire avantageusement l'épaisseur du plateau tibial 6 et, par conséquent, de minimiser l'épaisseur de la résection osseuse tibiale à opérer. Elle permet en outre de dégager la zone d'implantation du pion 8.

Le déport vers l'intérieur du pion 8, autour de l'axe duquel l'implant tibial 3 est destiné à pivoter, permet d'augmenter l'amplitude du mouvement antéro-postérieur de la zone de contact entre le condyle fémoral externe et la zone glénoïde externe et, par conséquent, de diminuer l'amplitude correspondante de la zone de contact fémoro-tibial interne, d'une manière qui correspond à l'articulation naturelle.

Le déport vers l'avant de l'axe du pion 8 par rapport à l'axe des tourillons 20 permet d'augmenter la stabilité de l'articulation au cours de son mouvement et d'assurer une amplitude de pivotement axial de l'implant tibial 3 qui est d'environ 30° tant en rotation interne qu'externe, ce mouvement se rapprochant de celui du mouvement naturel du tibia au cours de la flexion/extension de la jambe.

L'inclinaison de l'axe de la tige fémorale 10, et l'asymétrie des condyles assurent, au cours de la flexion de l'articulation du genou, une rotation du tibia vers l'intérieur, selon une amplitude proche de celle qui existe dans le mouvement naturel.

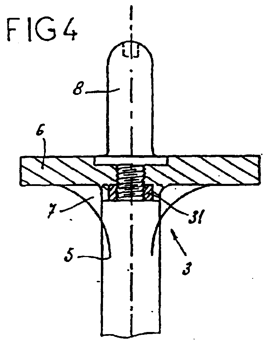

La figure 4 montre une variante de réalisation de la prothèse 2, dans laquelle le pion 8 n'est pas monobloc avec l'implant tibial 3 mais rapporté sur lui par vissage sur le plateau 6, avec un écrou de blocage 31.

## Revendications

1. Prothèse totale du genou, comprenant un implant tibial et un implant fémoral, l'implant tibial pouvant pivoter par rapport à l'implant fémoral selon, d'une part, un axe (C) sensiblement horizontal, afin de permettre le mouvement de flexion/extension de la jambe et, d'autre part, un axe vertical (A) qui, permettant un pivotement limité du tibia selon son axe, est constitué par un pion (8) monté avec possibilité de coulissement axial dans l'alésage qui lui sert de palier de rotation, l'axe vertical (A) selon lequel l'implant tibial (3) pivote étant, d'une part, déporté vers l'avant par rapport à l'axe horizontal (C) de flexion/extension de la jambe et, d'autre part, décalé vers l'intérieur par rapport à l'axe (B) médian longitudinal de l'implant tibial, la prothèse comprenant aussi une pièce centrale (16) d'assemblage des éléments tibial (3) et fémoral (4) réalisant le double déport précité, c'est-à-dire comprenant des moyens (17, 18) de guidage de l'élément tibial (3) par rapport à l'élément fémoral (4) selon deux axes non concourants, caractérisée en ce que l'implant tibial (3) comprend un pion (8) formant pivot, sur lequel peut être inséré un fourreau (15) en matière favorisant le glissement, et l'implant fémoral (4) reçoit dans sa cavité intercondylienne la pièce centrale d'assemblage (16),qui est percée d'une cavité borgne longitudinale (17), permettant son engagement sur le fourreau (15) et constituant un alésage servant de palier au pion (8), et qui comprend deux alésages horizontaux coaxiaux (18) ménagés dans ses faces latérales, destinés, lorsque la pièce (16) est engagée dans la cavité intercondylienne, à venir en coïncidence avec deux alésages coaxiaux (19) percés dans les parois latérales (12) de l'implant (4) délimitant ladite cavité (11) et à recevoir deux tourillons coaxiaux (20) formant pivot.

2. Prothèse selon la revendication 1, caractérisée en ce que l'axe (D) de la tige (10) de l'implant fémoral est incliné de haut en bas et de l'extérieur vers l'intérieur par rapport à l'axe longitudinal (B) du corps de l'implant tibial.

3. Prothèse selon l'une quelconque des revendications 1 et 2, caractérisée en ce que la gorge trochléenne est délimitée par deux condyles (12a, 12b), asymétriques, dont celui extérieur (12a) est plus proéminent que celui intérieur (12b).

4. Prothèse selon la revendication 1, caractérisée en ce que le déport vers l'avant de l'axe vertical (A) de pivotement du tibia est inférieur ou égal à 8 mm, tandis que son déport vers l'intérieur est inférieur ou égal à 10 mm.

5. Prothèse selon la revendication 2, caractérisés en ce que l'angle que forme l'axe (D) de la tige (10) de l'implant fémoral avec l'axe longitudinal (B) du corps de l'implant est de l'ordre de 4°.

6. Prothèse selon la revendication 1, caractérisée en ce que le fourreau (15) comprend un têton axial fileté (15a) destiné à être engagé au travers d'un trou lamé (25) aménagé dans la pièce (16) et à recevoir un écrou (26), pour son assemblage à la pièce (16).

7. Prothèse selon la revendication 1, caractérisée en ce que le fourreau (15) comprend une collerette (15a) du côté opposé à l'extrémité libre du pion (8), sur laquelle vient s'appuyer la pièce centrale d'assemblage (17).

8. Prothèse selon l'une des revendications 1 à 6, caractérisée en ce qu'elle comprend un implant rotulien (27) rapporté sur la rotule à la place de la partie réséquée de celle-ci et formant un patin glissant le long du bouclier trochléen (13) que forme l'implant fémoral (4).

9. Prothèse selon l'une des revendications 1 à 6, caractérisée en ce que l'implant tibial (3) comprend une tige (5) pour son ancrage et un plateau horizontal (6) dont la liaison avec la tige (5) est renforcée par deux nervures obliques (7) tournées vers l'arrière.

## Claims

1. A total knee prosthesis comprising a tibial implant and a femoral implant, the tibial implant being able to rotate relative to the femoral implant, on the one hand in a substantially horizontal axis (C) in order to enable a flection/extension movement of the leg and, on the other hand, in a vertical axis (A) that, enabling a limited rotation of the tibia according to its axis, is made up of a pin (8) mounted with possibility of axial sliding in a bore having the function of a rotation bearing for the same, the vertical axis (A) according to which the tibial implant (3) pivots being, on the one hand, offset to a forward position relative to the horizontal flection/extension axis (C) of the leg and, on the other hand, offset to an inward position relative to the longitudinal median axis (B) of the tibial implant, said prosthesis also comprising a central piece (16) for assembling the tibial (3) and femoral (4) elements carrying out said dual offsetting, i.e. comprising means (17, 18) for guiding the tibial element (3) relative to the femoral element (4) according to two non-concurrent axes, characterized in that the tibial implant (3) comprises a pivot pin (8) on which a sleeve (15) made of a sliding-promoting material is fitted, and the femoral implant (4) receives the central assembling piece (16) in its intercondyloid cavity, said assembling piece being provided with a longitudinal blind cavity (17) enabling engagement thereof on the sleeve (15) and constituting a boring to be used as a bearing for the pin (8), and comprising two horizontal coaxial bores (18) formed in its side faces, which, when the piece (16) is engaged in said intercondyloid cavity, are intended for coming into coincidence with two coaxial bores (19) formed in the side walls (12) of the implant (4) delimiting said cavity (11) and for receiving two coaxial pivot gudgeons.

2. A prosthesis as claimed in claim 1, characterized in that the axis (D) of the femoral implant rod (10) is inclined from top to bottom and from the outside to the inside relative to the longitudinal axis (B) of the tibial implant body.

3. A prosthesis as claimed in anyone of claims 1 and 2, characterized in that the trochlear throat is delimited by two asymmetric condyles (12a, 12b) the outer one (12a) of them being more jutting than the inner one (12b).

4. A prosthesis as claimed in claim 1, characterized in that forward offsetting of the vertical pivot axis (A) of the tibia is less than or equal to 8 mm, while its offsetting towards the inside is less than or equal to 10 mm.

5. A prosthesis as claimed in claim 2, characterized in that the angle formed by the axis (D) of the rod (10) of the femoral implant with the longitudinal axis (B) of the implant body is in the order of 4°.

6. A prosthesis as claimed in claim 1, characterized in that the sleeve (15) comprises a threaded axial stud (15a) intended for engagement through a hole (25) formed in the piece (16) and for receiving a nut (26), for assembling with the piece (16).

7. A prosthesis as claimed in claim 1, characterized in that the sleeve (15) comprises a flange (15a) on the opposite side relative to the free end of the pin (8), on which the central assembling piece (17) rests.

8. A prosthesis as claimed in one of claims 1 to 6, characterized in that it comprises a patellar implant (27) inserted in the patella or kneecap in place of the resected portion thereof and forming a runner sliding along the trochlear shield forming the femoral implant (4).

9. A prosthesis as claimed in one of claims 1 to 6, characterized in that the tibial implant (3) comprises a rod (5) for anchoring of same and a horizontal plate (6), connection of said plate with the rod (5) being reinforced by two oblique ribs (7) turned rearwardly.

## Patentansprüche

1. Ganzknieprothese, umfassend ein Schienbeinimplantat und ein Oberschenkelimplantat, wobei das Schienbeinimplantat gegenüber dem Oberschenkelimplantat einerseits gemäß einer im wesentlichen horizontalen Achse (C), um die Beuge-Streckbewegung des Beines zu ermöglichen, und andererseits gemäß einer vertikalen Achse (A) verschwenkbar ist, die bei einer begrenzten Verschwenkung des Schienbeines gemäß der eigenen Achse aus einem Bolzen (8) besteht, der mit axialer Gleitmöglichkeit im Sitz eingebracht ist, der ihm als Drehlager dient, wobei die vertikale Achse (A), gemäß der das Schienbeinimplantat (3) verschwenkt wird, einerseits gegenüber der horizontalen, Beuge-/Streckachse (C) des Beines außerachsig nach vorne liegt und andererseits gegenüber der Längsmittelachse (B) des Schienbeinimplantates nach innen versetzt ist, wobei die Prothese auch ein mittleres Zusammenbauteil (16) des Schienbeinimplantates (3) und des Oberschenkelimplantates (4) umfasst, das die doppelte Außerachsigkeit verwirklicht, d.h. Führungsmittel (17, 18) des Schienbeinimplantates (3) gegenüber dem Oberschenkelimplantates (4) gemäß zwei nicht zusammenlaufenden Achsen umfasst, dadurch gekennzeichnet, dass das Schienbeinimplantat (3) einen Anlenkzapfen (8) umfasst, auf dem eine Muffe (15) aus einem Material aufgeschoben werden kann, das die Gleitfähigkeit erleichtert und dass das Oberschenkelimplantat (4) in seiner Interkondilenhöhlung das mittige Zusammenbauteil (16) aufnimmt, in dem eine Längssackhöhlung (17) ausgenommen wird, die dessen Eingriff an der Muffe (15) erlaubt und eine Aufnahme bildet, die als Lager für den Anlenkbolzen (8) dient und zwei koaxiale, in seinen Seitenflanken ausgearbeiteten Bohrungen (18) umfasst, die dazu bestimmt sind, wenn das Zusammenbauteil (18) in der Interkondylenhöhlung eingreift, mit den beiden koaxialen Bohrungen (19) zusammenzufallen, die in den Seitenwänden (12) des Oberschenkelimplantates (4) ausgenommen sind, welche die Höhlung (11) begrenzen, und beide koaxiale Anlenkbolzen (20) aufnehmen.

2. Prothese nach Anspruch 1, dadurch gekennzeichnet, dass die Achse (D) der Stange (10) des Oberschenkelimplantates von oben nach unten und von außen nach innen gegenüber der Längsachse (B) des Körpers des Schienbeinimplantates geneigt ist.

3. Prothese nach einem beliebigen der Ansprüche 1 und 2, dadurch gekennzeichnet, dass die Trochleakehle durch zwei asymmetrische Kondylen (12a, 12b) begrenzt ist, von denen der äußere (12a) gegenüber dem inneren (12b) vorstehender ist.

4. Prothese nach Anspruch 1, dadurch gekennzeichnet, dass die Außerachsigkeit nach vorne der vertikalen Anlenkachse (A) des Schienbeines kleiner oder gleich 8 mm ist, während seine Außerachsigkeit nach innen kleiner oder gleich 10 mm ist.

5. Prothese nach Anspruch 2, dadurch gekennzeichnet, dass der zwischen der Achse (d) der Stange (10) des Oberschenkelimplantates und der Längsachse (B) des Körpers des Implantates gebildete Winkel in der Größenordnung von 4° liegt.

6. Prothese nach Anspruch 1, dadurch gekennzeichnet, dass die Muffe (5) einen axialen Gewindezahn (15a) umfasst, der dazu bestimmt ist, über eine im Teil (16) ausgenommene, angesenkte Bohrung (25) in Eingriff zu kommen und eine Schraubmutter (26) für dessen Zusammenbau mit dem Teil (16) aufzunehmen.

7. Prothese nach Anspruch 1, dadurch gekennzeichnet, dass die Muffe (15) einen Kragen (15a) auf der dem freien Ende des Bolzens (8) abgewandten Ende umfasst, auf dem das mittige Zusammenbauteil (17) in Anlage kommt.

8. Prothese nach einem der Ansprüche von 1 bis 6, dadurch gekennzeichnet, dass sie auf der Kniescheibe ein an der Stelle des von derselben resezierten Teils aufgebrachtes Kniescheibenimplantat (27) umfasst, das einen Gleitschuh bildet, der längs des Trochleaschildes (13) gleitet, was das Oberschenkelimplantat (4) bildet.

9. Prothese nach einem der Ansprüche von 1 bis 6, dadurch gekennzeichnet, dass das Schienbeinimplantat (3) eine Stange (5) für dessen Verankerung und eine horizontale Platte (6) umfasst, deren Vereinigung mit der Stange (5) durch zwei schräge, nach hinten gerichtete Stege (7) verstärkt ist.
